# EUROPEAN PATENT APPLICATION

(11) **EP 1 930 480 A1**
(43) Date of publication of application: **11.06.2008**
(21) Application number: 06782458.1
(22) Date of filing: 08.08.2006
(51) Int. Cl.: C25D 11/26, A61C 5/02, A61C 7/20, A61L 29/00, C22C 19/03, A61F 2/84, A61M 25/00

(54) **TITANIUM-NICKEL ALLOY, METHOD FOR MODIFYING TITANIUM-NICKEL ALLOY SURFACE, BIOCOMPATIBLE MATERIAL**

(30) Priority: 10.08.2005 JP 2005232502; 31.03.2006 JP 2006098016
(71) Applicant: National University Corporation Tokyo Medical and Dental University, Tokyo 113-8510 (JP)
(72) Inventor: YONEYAMA, Takayuki, Tokyo 113-8510 (JP); FUKUSHIMA, Osamu, Tokyo 135-0085 (JP); HANAWA, Takao, Tokyo 113-8510 (JP); DOI, Hisashi, Tokyo 113-8510 (JP)
(74) Representative: Matthews, Derek Peter
(86) International application number: PCT/JP2006/315616
(87) International publication number: WO 2007/018189

(57) **Abstract**

Provided is a titanium-nickel alloy improved in corrosion resistance due to the reduced Ni content in the surface layer.

Since a film (modified layer) extremely reduced in Ni concentration compared to the interior can be formed on the surface of a titanium-nickel alloy, by applying electrolysis treatment in a properly controlled electrolytic solution, the corrosion resistance of the alloy is improved. Specific examples of the electrolytic solution may include a mixture of glycerol, lactic acid and water (H₂O).

## Description

### Technical Field

The present invention relates to a titanium-nickel alloy having a reduced Ni content in the surface and improved in corrosion resistance, a method for modifying the surface of the titanium-nickel alloy and a biocompatible material using the titanium-nickel alloy.

### Background Art

An alloy containing titanium (Ti) and nickel (Ni) in an atomic ratio of about 1:1 (more specifically, the molar ratio of them mutually falls within the range of 48.5 to 51.5%) serves as a shape memory alloy. The alloy is known to have a superelastic effect, by which the alloy immediately regains its original shape only by releasing force after deformed, and titanium has excellent corrosion resistance and biocompatibility.

There have been attempts to use the features of the titanium-nickel alloy in medical materials. For example, Patent Document 1 discloses that a nickel-titanium alloy is used as a material for a mesh stent, which is inserted into a blood vessel and then enlarged in diameter, thereby inhibiting contraction of the blood vessel. Patent Documents 2 and 3 disclose that a surface treatment is provided to reduce the nickel concentration in the surface oxide film on a titanium-nickel alloy.

Furthermore, Non-Patent Document 1 describes that nickel of a titanium-nickel alloy and austenitic stainless steel is doubted as a causative agent for metal-allergy. The document further proposes, as a means for solving the problem, bringing the alloy into contact with nitrogen gas for 2 hours in a heat treatment furnace heated to 1200°C, thereby permitting the alloy to absorb about 1% by mass of nitrogen. Non-Patent Document 1 describes that mechanical strength and corrosion resistance are improved by nitrogen in place of nickel.

Patent Document 1: National Publication of International Patent Application No. 2001-516260
Patent Document 2: US Application 2004/0117001
Patent Document 3: WO2004/108983
Non-Patent Document 1: Research paper "Initiation of co-development of a less allergic dental material at low cost (Independent Administrative Institute, National Institute for Materials Science, December 4, 2003).

A titanium-nickel alloy exhibiting superelasticity has mechanical properties preferable as an orthodontic wire, stent or catheter; however, it contains nickel harmful to a living body in about a half amount by a molar ratio, as described above. The titanium-nickel alloy has a further problem in that compared to Ti-6Al-4V, which is known as an alloy for surgical implantation, the titanium-nickel alloy releases a large amount of ions in an aqueous solution.

Patent Document 1 provides no specific solutions for harmfulness of nickel to the living body. As a method for modifying the surface of a titanium-nickel alloy, various surface treatment methods are disclosed in Patent Document 2 and an ion doping method in Patent Document 3. However, an alloy improved in corrosion resistance, which is the most important feature of a medical material, regarding safeness, has not yet been found. Non-Patent Document 1 is concerned with harmfulness of nickel to the living body; however, an attempt to allow an alloy to absorb about 1 mass % of nitrogen is made with respect to austenitic stainless steel alone. There are no disclosures as to a titanium-nickel alloy.

### Disclosure of the Invention

To solve the aforementioned problems, the present invention is directed to provide a titanium-nickel alloy having a composition in which titanium and nickel are contained mutually in a molar ratio of 48.5 to 51.5%, characterized in that a modified layer reduced in nickel content compared to the interior is formed on the surface. More specifically, the ratio of nickel atoms to a single titanium atom of the modified layer can be set at 0.1 or less and was confirmed that the ratio can be reduced to less than 0.01.

The modified layer is preferably as thick as possible in consideration of, for example, harmfulness to the living body; however, a modified layer excessively thick is not desirable in consideration of deformation involved in shape memory effect and superelasticity. The modified layer could be formed with a thickness of 2.0 nm or more. The thickness of the modified layer can be changed by varying electrolysis conditions.

As means for forming a modified layer on the surface of the titanium-nickel alloy, for example, an electrolytic treatment performed in an electrolytic solution containing weak acid may be mentioned. More specifically, the electrolytic solution is preferably constituted of 10 to 40 vol % of glycerol, 0.1 to 80 vol % of lactic acid and water (the remainder).

A titanium-nickel alloy according to the present invention can be used in a biocompatible material (a material in contact with the living body when used and less harmful to the living body from allergic and carcinogenic points of view).
Examples of the biocompatible material may include medical equipment, cosmetic tools, barber tools, cloth, personal belongings, general-purpose parts, accessories of personal belongings and general purpose parts, tableware, cookware, beverage tools, utensil, household hygiene goods, hobby/amusement goods, pet-accessory, sports goods, toys, portable watches such as a wristwatch, portable electronic equipment such as a handy phone and food processing machines.
Examples of the medical equipment may include diagnostic equipment, surgical equipment, treatment equipment and dental equipment.
As the surgical equipment and treatment equipment, mention may be made of a stent or a catheter. As the dental equipment, mention may be made of orthodontic wire or an endodontic file.

A titanium-nickel alloy according to the present invention is improved in corrosion resistance, since a modified layer, which is reduced in nickel content compared to the interior, is formed on the surface. Therefore, the titanium-nickel alloy is further improved in biocompatibility while maintaining the shape memory effect and superelasticity thereof.

Orthodontic wire, an endodontic file, a stent or a catheter can be manufactured from a titanium-nickel alloy according to the present invention by electrolysis under predetermined conditions. Therefore, even if a product has a complicated figure, it can be manufactured without any problem.

### Brief Description of the Drawings

Figure 1 (a) is a graph showing the relationship between the number of immersion days in 1% lactic acid and the amount of released Ti ions with respect to individual types of electrolytic solutions; and Figure (b) is a graph showing the relationship between the number of immersion days in 1% lactic acid and the amount of released Ni ions with respect to individual types of electrolytic solutions;
Figure 2 is a graph showing the amount (molar ratio) of Ti ions released in 1% lactic acid after a lapse of 30 days in comparison with that of Ni ions;
Figure 3 is a graph showing anode polarization curves of titanium-nickel alloys treated with different methods in a 0.9% aqueous NaCl solution;
Figure 4 is a graph showing 2P spectra of Ti by X-ray photoelectron spectroscopy (XPS) with respect to individual types of electrolytic solutions;
Figure 5 is a graph showing 2P spectra of Ni by X-ray photoelectron spectroscopy (XPS) with respect to individual types of electrolytic solutions;
Figure 6 is a graph showing 2P spectra of Ti by X-ray photoelectron spectroscopy (XPS) when the composition ratio of GLW is varied;
Figure 7 is a graph showing 2P spectra of Ni by X-ray photoelectron spectroscopy (XPS) when the composition ratio of GLW is varied;
Figure 8 is a graph showing the results of composition analysis by Auger electron spectroscopy (AES) of a modified layer in the case where the content of lactic acid of GLW is set at 10 vol %;
Figure 9 is a graph showing the results of composition analysis by Auger electron spectroscopy (AES) of a modified layer in the case where the content of lactic acid of GLW is set at 20 vol %;
Figure 10 is a graph showing the results of composition analysis by Auger electron spectroscopy (AES) of a modified layer in the case where the content of lactic acid of GLW is.set at 80 vol %;
Figure 11 is a graph showing the results of composition analysis by Auger electron spectroscopy (AES) of the surface layer of a sample mechanically polished;
Figure 12 is a graph showing the relationship between the photoelectron take-off angle, effective photoelectron escape depth and Ti content; and

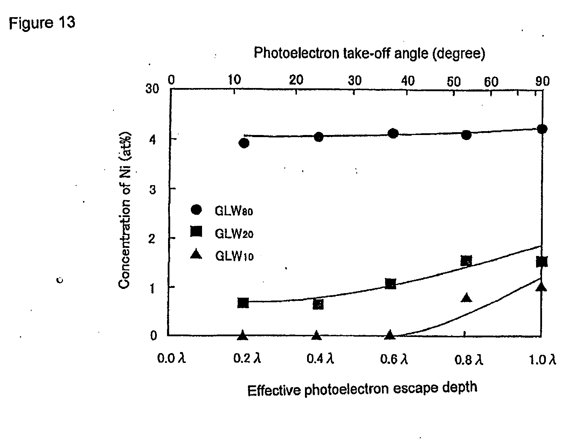

Figure 13 is a graph showing the relationship between the photoelectron take-off angle, effective photoelectron escape depth and Ni content.

### Best Mode for Carrying out the Invention

The present inventors had a finding that the amount of ions released from a titanium-nickel alloy is suppressed by applying electrolysis treatment to the titanium-nickel alloy. Then, they applied electrolysis treatment by use of a plurality of types of electrolytic solutions prepared by varying the composition to check corrosion resistance.

### (Material and method)

As sample materials, discs (8 mm diameter, 1 mm thick) of a titanium-nickel alloy (NT-E4, manufactured by The Furukawa Electric Co., Ltd, Ti-50.85 mol%Ni) were used. The discs were mechanically polished to obtain mirror surfaces, subjected to electrolytic treatment under four conditions using the electrolytic solutions different in composition as shown in the following Table 1 (GLS, GLW, GP and HP) and further washed ultrasonically with acetone, ethyl alcohol and ultrapure water to prepare test pieces. A sample piece subjected to mechanical polishing alone was used as a control.
In an immersion test, test pieces were fully immersed in a 20 ml of 1.0% lactic acid poured in a polyethylene container and shaken at 1.67 Hz and 37°C.
After each of the test pieces was immersed for 0.63 and 2.6 Ms (corresponding to 7 and 30 days, respectively), the immersion solution was analyzed by an ICP emission spectrometer (JICP-PS3000UV, Leeman Labs) to quantitatively determine the concentration of ions released in the solution. In this manner, the amount of ions released from each of the sample pieces per unit area was obtained.
In the anode polarization test, the test pieces were each covered with a masking tape having pores of 3 mm in diameter and used as samples. The test was performed by placing samples in a 0.9% aqueous NaCl solution (37°C) and applying a voltage up to 1600 mV by use of an automatic polarization measurement apparatus (HZ-1A, manufactured by Hokuto Denko Corporation) and using a saturated calomel electrode as a reference electrode, at a trace speed of 2 mV/s.

**[Table 1]**

| Treatment | Electrolyte Component/Amount | | Anodic current /voltage | Time (min) |
|---|---|---|---|---|
| GLS | Glycerol (84~87%) | 50 ml | 0.4A/10V | 15 |
| | C₂H₅OH (99.5%) | 50 ml | | |
| | Lactic acid (90%) | 30 ml | | |
| | H₂SO₄ (>96%) | 10 ml | | |
| GLW | Glycerol (84~87%) | 50 ml | unnoticeable/50V | 30 |
| | Lactic acid(90%) | 50 ml | | |
| | H₂O | 20 ml | | |
| GP | Glycerol (84~87%) | 50 ml | unnoticeable/25V | 15 |
| | Phosphoric acid (85%) | 40 ml | | |
| | C₂H₅OH (99.5%) | 50 ml | | |
| HP | Hydrogen peroxide (35%) | 50 ml | 0.4A/35V | 15 |
| | Phosphoric acid (85%) | 5 ml | | |
| MP | Control (mechanical polishing) | | - | - |

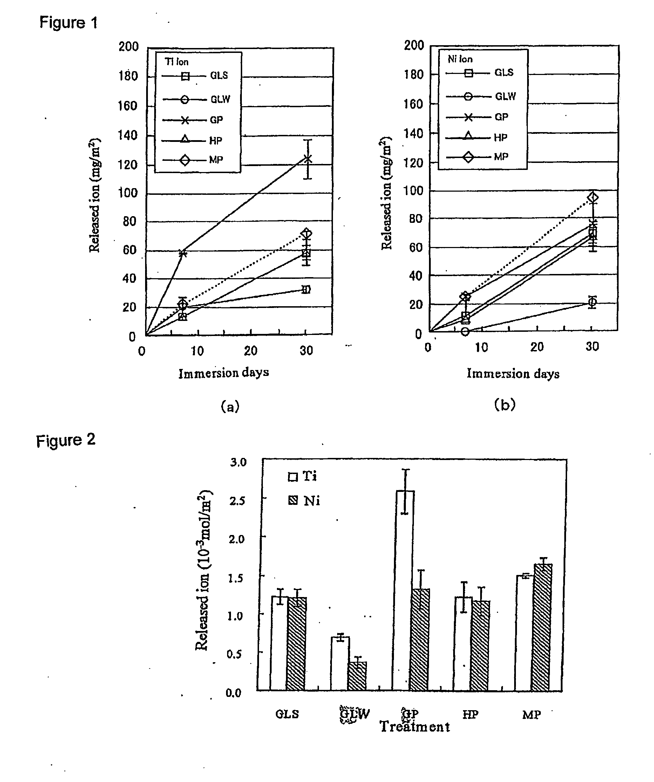

The results of the immersion test mentioned above are shown in Figures 1 and 2. Figure 1(a) is a graph showing the relationship between the number of immersion days in an electrolytic solution and the amount of released Ti ions with respect to individual types of electrolytic solutions; and Figure 1(b) is a graph showing the relationship between the number of immersion days in an electrolytic solution and the amount of released Ni ions with respect to individual types of electrolytic solutions. Figure 2 is a graph showing the amount (molar ratio) of Ti ions released in each of the electrolytic solutions after a lapse of 30 days in comparison with that ofNi ions.

From Figures 1 and 2, the following were found. First, the amounts of ions released from the samples of GLS, GLW and HP treatments excluding GP treatment were low compared to the sample of MP treatment in which no electrolysis is performed. From this, GLS, GLW and HP treatments are considered to be effective for suppressing release of ions. In particular, in the sample of GLW treatment, effect of suppressing release of ions was significant (see Figure 1).

The amount (in terms of molar ratio) of released ions, more specifically, the amount of Ti ions released from the samples of GP and GLW treatments significantly exceeded over the amount of Ni ions. In particular, in the sample of GLW treatment, the amount of released Ti ions was suppressed to about 1/2 of that of the sample of MP treatment; and the amount of Ni ions was suppressed to about 1/4 thereof. The corrosion resistance of the sample was significantly improved (see Figure 2).
From this, the surface state of the sample of the GLW treatment is considered to be different from those of other samples from which Ti ions and Ni ions are repeated in almost the same amounts. In other words, it is expected that the release of ions can be further suppressed by varying the composition of the electrolytic solution for electrolysis treatment. Furthermore, the surface is conceivably modified in various ways depending upon the compositions of electrolytic solutions.

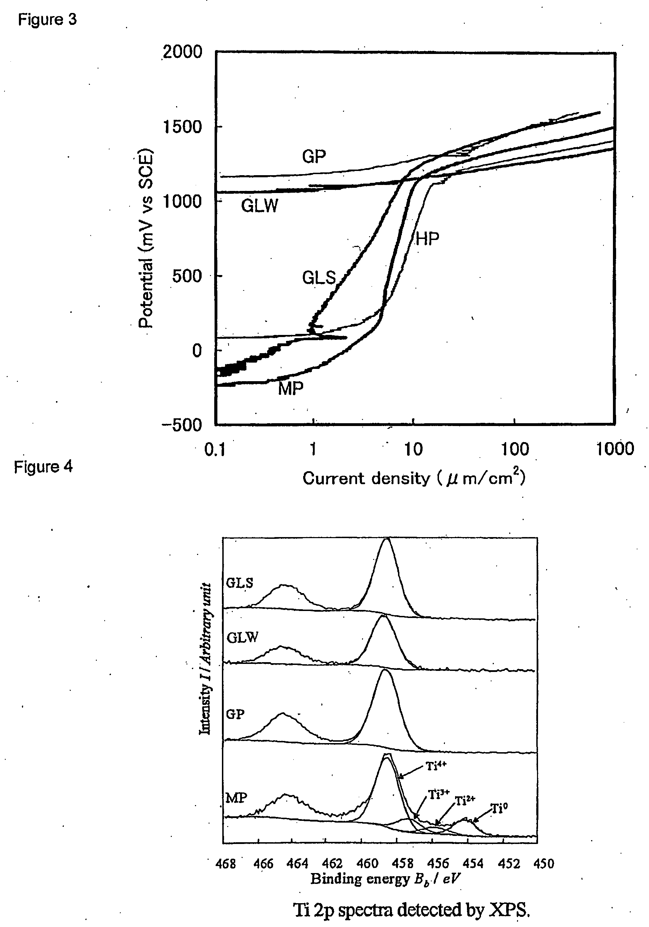

The results of the anode polarization test are shown in Figure 3. Generally, in the anode polarization test, it is evaluated that the higher the spontaneous corrosion potential at the time no current flows; the lower the density of the current (passivation current) at the time the increase of current reaches a state of equilibrium; the higher the pitting corrosion potential at which a passive film is broken and a large amount of current abruptly flows, the more excellent the corrosion resistance is.

From Figure 3, the following are found. The break-down due to pitting corrosion occurrence was not observed in any one of the samples. Relatively good corrosion resistance was shown. Note that current gradually increases at a voltage of 1200 to 1300 mV not because pitting corrosion occurs but because oxygen generates. In the GLS treatment, a passivation current density slightly decreases compared to the MP treatment. In the HP treatment, since a spontaneous corrosion potential was higher by about 300 mV, the corrosion resistance was slightly improved. In the samples of GLW and GP treatments, the spontaneous corrosion potential was as high as 1000 mV or more. Corrosion resistance was greatly improved.

Apparent from the results of the immersion test and the anodic polarization test, the corrosion resistance of a titanium-nickel alloy treated with GLW was significantly improved compared to that treated with MP.

Then, electrolysis was performed using the electrolytic solutions having the compositions shown in the following Table 2. The surface of a sample was analyzed by X-ray photoelectron spectroscopy (XPS) to determine the thickness and chemical structure of the surface oxidation film. The analysis conditions are as follows.

**[Table 2]**

| (vol%) | | | | | |
|---|---|---|---|---|---|
| GLS | | GLW | | GP | |
| Glycerol | 36 | Glycerol | 42 | Glycerol | 36 |
| Lactic acid | 21 | Lactic acid | 42 | H₃PO₄ | 28 |
| H₂SO₄ | 7 | H₂O | 16 | C₂H₅OH | 36 |
| C₂H₅OH | 36 | | | | |

Discs (8 mm diameter, 1 mm thick) of a titanium-nickel alloy (NT-E4, manufactured by The Furukawa Electric Co., Ltd, Ti-50.85 mol%Ni) were mechanically polished to obtain mirror-finished surfaces and subjected to electrolytic treatment using three types of electrolytic solutions (GLS, GLW and GP), whose compositions are shown above (Table 2). In addition, a disc (MP) to which no electrolysis was performed was prepared. In total, four types of samples were prepared.

The surface oxidation films of these four types of samples were checked for thickness and chemical structure. The apparatus used herein was an X-ray photoelectron spectroscopic apparatus (SSI-SSX-100). The irradiation X-ray was Al-Kα beams (hv=1486.61 eV). Analysis was made at a photoelectron escape angle of 35°.

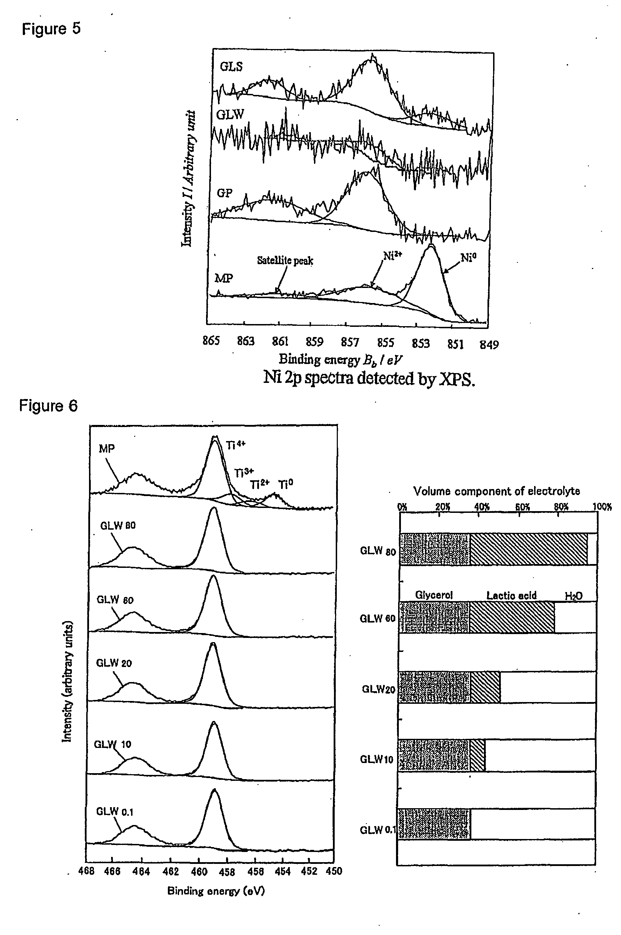

As a result of the analysis, oxidation of Ti of the surface oxidation film was accelerated by electrolysis treatment. Most of the Ti ions were converted to tetravalent Ti ions (see Figure 4).
With respect to Ni, the ratio of divalent Ni increased. In GLW, the intensity of the Ni spectrum decreased (see Figure 5).

Furthermore, as is shown in Table 3 below, the thickness of the surface oxidation film (surface layer) was increased by electrolysis treatment and the composition ratio of Ni of the film was decreased.

**[Table 3]**

| | | |
|---|---|---|
| Treatment | t(nm) | Chemical composition |
| GLS | 4.1 | TiNi_{0.55} O_{2.66} (OH)_{1.34} (SO₄)_{0.06} • 0.58H₂O |
| GLW | 5.5 | TiNi_{0.06} O_{1.75} (OH)_{1.48} • 0.72H₂O |
| GP | * | TiNi_{0.29} O_{1.51} (OH)_{2.12} (PO₄)_{0.36} • 0.95H₂O |
| MP | 2.1 | TiNi_{0.79} O_{2.58} (OH)_{2.71} • 0.73H₂O |

| | | |
|---|---|---|
| * The thickness of a surface oxidation film is the measurable limit or more | | |

From the above, it was found that a film (modified layer) whose Ni concentration is extremely lower than that of the interior can be formed on the surface of a titanium-nickel alloy by electrolysis treatment using an appropriately controlled electrolytic solution. Then, to determine the optimum conditions, GLW was chosen as an electrolytic solution and XPS analysis was performed by varying the ratio of the components consisting of GLW, that is, glycerol, lactic acid and water (H₂O).

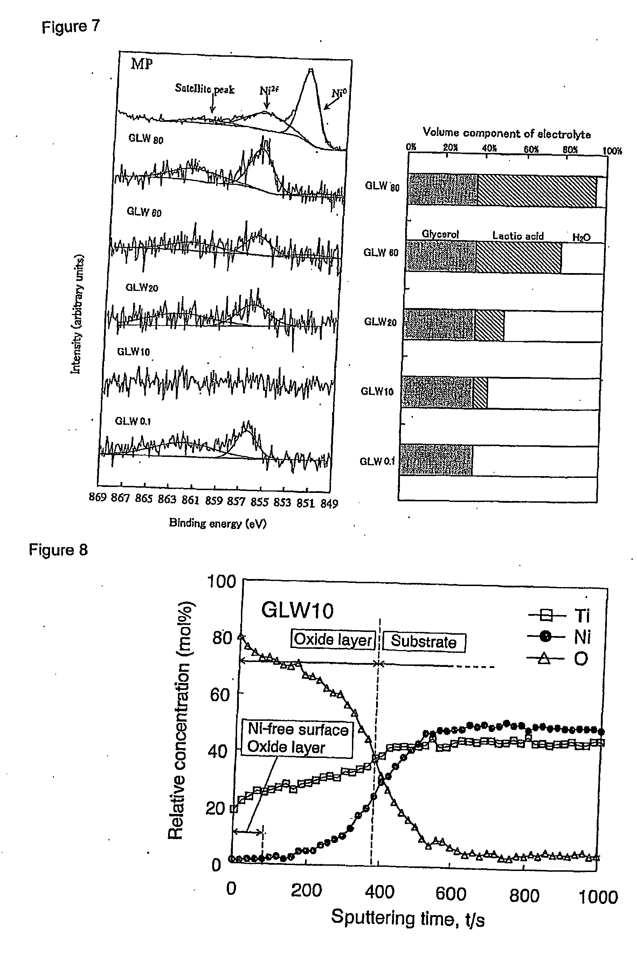

Figures 6 and 7 show the results of the analysis mentioned above. Oxidation of Ti proceeds almost regardless of the content of lactic acid and mostly changes into tetravalent Ti, as shown in Figure 6. On the other hand, Ni was found to decrease, as shown in Figure 7, when the content of glycerol is 35 vol % and the content of lactic acid is 0.1 vol % to 20 vol % and extremely decreases, particularly when the content of lactic acid is 10 vol %. More specifically, in the sample MP, to which mechanical polishing alone was applied, the integrated intensity of a Ni⁰ peak derived from the underlying metal was large, whereas no Ni⁰ peak was detected in GLW80, GLW60, GLW20, GLW10 and GLW0.1, to which electrolysis treatment was applied. Furthermore, in GLW10, a Ni²⁺ peak was not detected, either. From this, an oxidation film containing no Ni is conceivably formed on the surface of a Ti-Ni alloy. This sample was analyzed by changing a photoelectron escape angle. As a result, no Ni peaks were detected to the depth, which corresponds to 60% of the photoelectron escape depth.

Note that, the graphs of Figures 6 and 7 show the case where the content of glycerol was set at 35 vol %. However, the same tendency was obtained as long as the content of glycerol falls within the range of 10 vol % to 40 vol %.

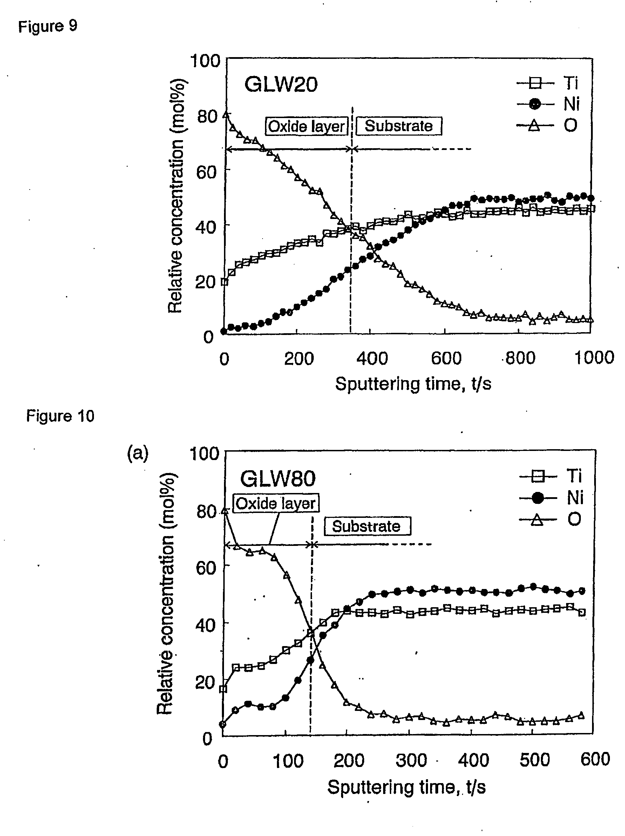

Furthermore, Figures 8 to 11 are the graphs showing the results of composition analysis of the surface layers of samples of GLW where contents of lactic acid are 10 vol %, 20 vol % and 80 vol % and of a sample mechanically polished, by Auger electron spectroscopy (AES). As is apparent also from these graphs, the composition ratio ofNi contained in the surface layer (oxide film) can be reduced by choosing GLW composed of glycerol, lactic acid and water (H₂O) as an electrolyte. In particular, an electrolyte GLW containing 10 vol % of lactic acid is the most effective.

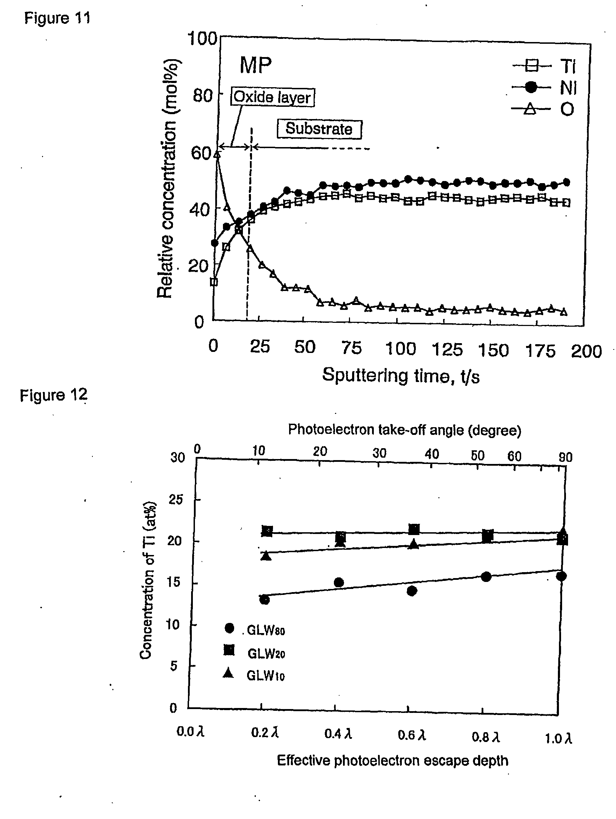

Figure 12 (13) is the graph showing the relationship between photoelectron take-off angle, effective photoelectron escape depth and Ti content (Ni content). It was found that the concentration ofNi ions extremely decreases in the case where the content of lactic acid is 10 vol % to 20 vol % compared to 80 vol %.

Furthermore, Table 4 indicate the contents of individual components of the film when the content of lactic acid was set at 0.1 vol %, 10 vol %, 20 vol %, 60 vol % and 80 vol %. Also in GLW80, the atomic ratio ofTi and Ni was 1:0.16, and in GLW10, Ni was not detected.

**[Table 4]**

| Treatment | t(nm) | Chemical composition |
|---|---|---|
| GLW80 | * | TiNi_{0.16} O_{1.79} (OH)_{1.72} • 0.49H₂O |
| GLW60 | * | TiNi_{0.06} O_{1.92} (OH)_{1.95} • 0.71H₂O |
| GLW20 | * | TiNi_{0.07} O_{1.87} (OH)_{1.28} • 0.36H₂O |
| GLW10 | * | TiNi_{0.00} O_{1.98} (OH)_{1.40} • 0.37H₂O |
| GLW0.1 | * | TiNi_{0.15} O_{1.72} (OH)_{1.75} • 0.76H₂O |

| | | |
|---|---|---|
| * The thickness of a surface oxidation film is the measurable limit or more | | |

## Claims

1. A titanium-nickel alloy having a composition in which titanium and nickel are contained mutually in a molar ratio of 48.5 to 51.5%, **characterized in that** a modified layer reduced in nickel content compared to the interior is formed on the surface, thereby improving corrosion resistance.

2. The titanium-nickel alloy according to claim 1, **characterized in that** the ratio of nickel atoms relative to a single titanium atom of the modified layer is 0.16 or less.

3. The titanium-nickel alloy according to claim 1 or 2, **characterized in that** the thickness of the modified layer is 2 nm or more.

4. A method for modifying the surface of a titanium-nickel alloy, **characterized by** electrolyzing a titanium-nickel alloy having a composition in which titanium and nickel are contained mutually in a molar ratio of 48.5 to 51.5%, in an electrolytic solution containing weak acid to form a modified layer reduced in nickel content compared to the interior on the surface.

5. The method for modifying the surface of a titanium-nickel alloy according to claim 4, **characterized in that** the electrolytic solution is composed of 10 vol % to 40 vol % of glycerol, 0.1 vol % to 80 vol % of lactic acid and water (the remainder).

6. A biocompatible material **characterized by** using the titanium-nickel alloy according to any one of claims 1 to 3.

7. The biocompatible material according to claim 6, **characterized in that** the biocompatible material is any one of medical equipment, cosmetic tools, barber tools, cloth, personal belongings, general-purpose parts, accessories of personal belongings and general purpose parts, tableware, cookware, beverage tools, utensil, household hygiene goods, hobby/amusement goods, pet-accessory, sports goods, toys, portable watches such as a wristwatch, portable electronic equipment such as a handy phone and food processing machines.

8. The biocompatible material according to claim 7, **characterized in that** the medical equipment is any one of diagnostic equipment, surgical equipment, treatment equipment and dental equipment.

9. The biocompatible material according to claim 8, **characterized in that** the surgical equipment and treatment equipment are a stent or a catheter.

10. The biocompatible material according to claim 8, **characterized in that** the dental equipment is orthodontic wire or an endodontic file.
